# EUROPÄISCHE PATENTSCHRIFT

(11) **EP 0 834 491 B1**
(45) Veröffentlichungstag und Bekanntmachung des Hinweises auf die Patenterteilung: **05.09.2001**
(21) Anmeldenummer: 97116709.3
(22) Anmeldetag: 25.09.1997
(51) Int. Cl.: C07C 13/28, C07C 43/188, C09K 19/30

(54) **Bisalkenylbicyclohexane und flüssigkristallines Medium**
Bisalkenyl bicyclohexane and liquid crystal medium
Bisalkenyle de bicyclohexane et milieu liquide cristallin

(30) Priorität: 02.10.1996 DE 19640851; 27.02.1997 DE 19707950
(43) Veröffentlichungstag der Anmeldung: 08.04.1998
(73) Patentinhaber: MERCK PATENT GmbH, 64293 Darmstadt (DE)
(72) Erfinder: Reiffenrath, Volker, 64380 Rossdorf (DE); Hirschmann, Harald, Dr., 64291 Darmstadt (DE)

(56) Entgegenhaltungen:
- EP-A- 0 168 683
- EP-A- 0 427 957
- EP-A- 0 750 028
- DATABASE WPI Section Ch, Week 9722 Derwent Publications Ltd., London, GB; Class E15, AN 97-241667 XP002051962 & JP 09 077 692 A (DAINIPPON INK & CHEM INC) , 25.März 1997
- KELLY S M ET AL: "THE SYNTHESIS AND LIQUID CRYSTAL TRANSITION TEMPERATURES OF SOME WEAKLY POLAR NEMATIC METHYL (E)-(TRANS-4-SUBSTITUTED-CYCLOHEXYL)- ALLYL ETHERS" LIQUID CRYSTALS, Bd. 16, Nr. 3, 1.März 1994, Seiten 491-507, XP000434165

## Beschreibung

Die Erfindung betrifft Bisalkenylbicyclohexane der Formel I, 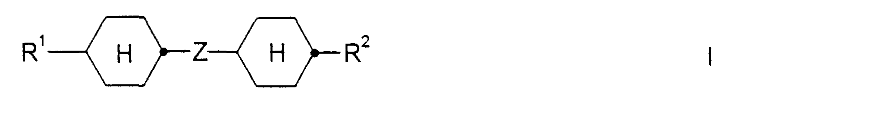 worin
- R¹ und R²: jeweils einen Alkenylrest mit 2 bis 7 C-Atomen, und
- Z: -CH₂CH₂-, -OCH₂-, -CH₂O-, oder eine Einfachbindung
bedeuten.

Die Erfindung betrifft weiterhin die Verwendung dieser Verbindungen als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Medien geeignet sind und insbesondere gleichzeitig eine vergleichsweise geringe Viskosität besitzen sowie eine relativ hohe Nematogenität.

Im Hinblick auf die verschiedensten Einsatzbereiche derartiger Verbindungen mit niedrigem Δn und günstigen Werten für die Viskosität war es jedoch wünschenswert, weitere Verbindungen mit hoher Nematogenität zur

Verfügung zu haben, die auf die jeweiligen Anwendungen genau maßgeschneiderte Eigenschaften aufweisen. Mit Hilfe der erfindungsgemäßen Verbindungen lassen sich STN-Mischungen mit überraschend guter Steilheit realisieren. Im Vergleich zu den entsprechenden dialkylierten Verbindungen und Monoalkenylverbindungen weisen diese Substanzen höhere Klärpunkte, eine bessere Löslichkeit, sowie eine wesentlich geringere Tendenz zur Ausbildung smektischer Phasen auf.

Verbindungen der Formel I werden von den breiten Formeln der DE-OS 42 11 694, DE-OS 44 146 47, EP 0 427 957 und EP 0 168 683 umfaßt, aber dort nicht genannt. Weder die Herstellung der erfindungsgemäßen Verbindungen noch die besonderen Eigenschaften werden dort beschrieben. EP 0 750 028 beschreibt Alkenylbicyclohexanderivate mit Butyl- und Butenylbrücken.

Es wurde nun gefunden, daß Verbindungen der Formel I als Komponenten flüssigkristalliner Medien vorzüglich geeignet sind. Insbesondere verfügen sie über vergleichsweise niedrige Viskositäten. Mit ihrer Hilfe lassen sich stabile flüssigkristalline Medien mit breitem nematischen Mesophasenbereich und vorteilhaften Werten für die optische und dielektrische Anisotropie erhalten. Diese Medien weisen ferner ein sehr gutes Tieftemperaturverhalten auf.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektroopische Anzeigeelemente, die derartige Medien enthalten.

Bevorzugte Verbindungen fallen unter die Formeln IA, IB und IC:

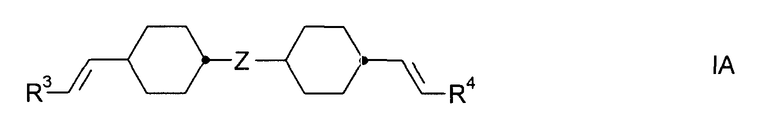

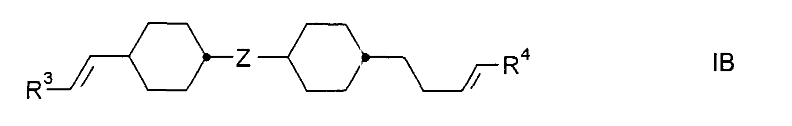

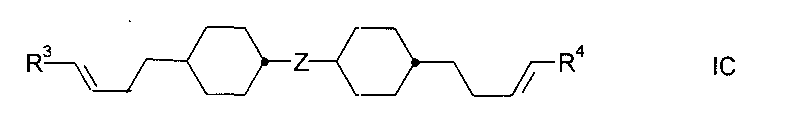

R³ und R⁴ bedeuten jeweils unabhängig voneinander H oder einen Alkylrest mit 1 bis 5C-Atomen, vorzugsweise H, CH₃ oder C₂H₅, ferner n-C₃H₇.

Eine ganz besonders bevorzugte kleinere Gruppe von Verbindungen ist diejenige der Teilformeln I1 bis I21:

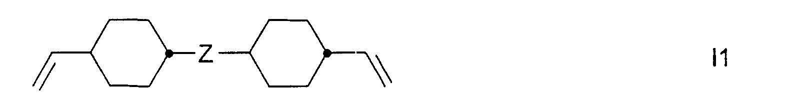

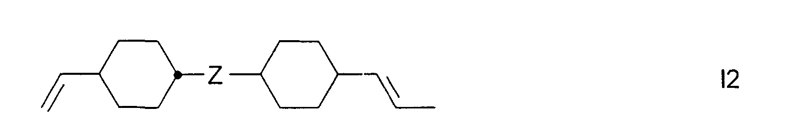

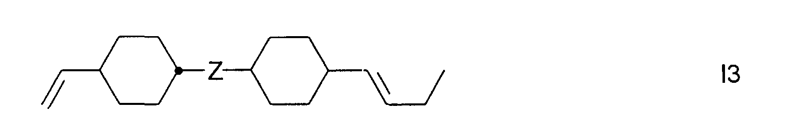

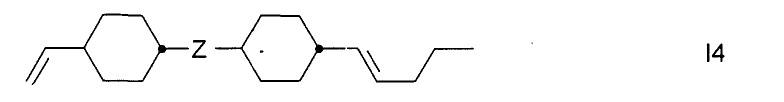

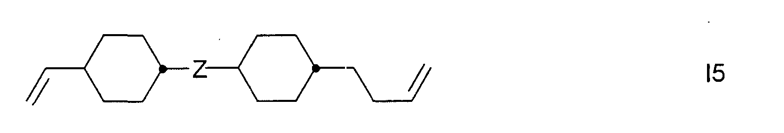

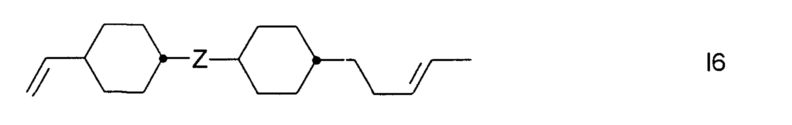

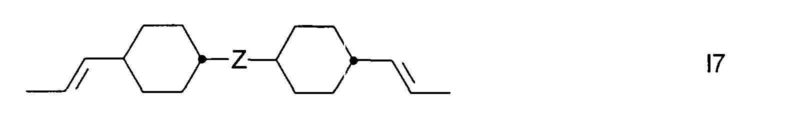

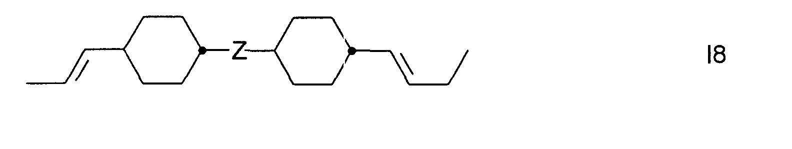

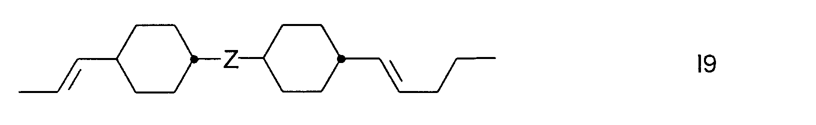

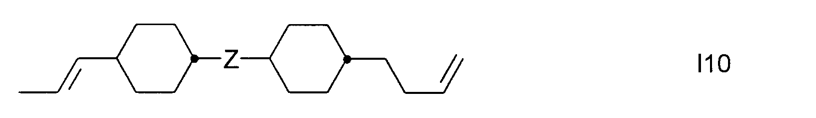

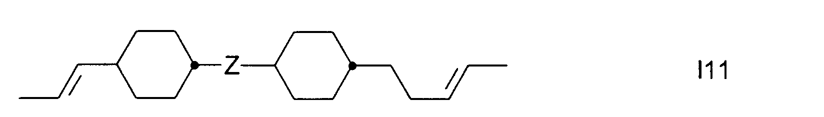

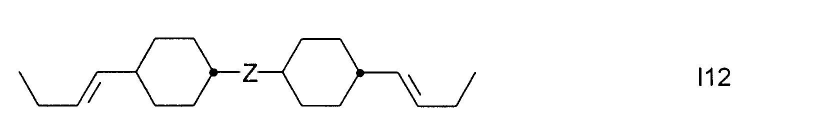

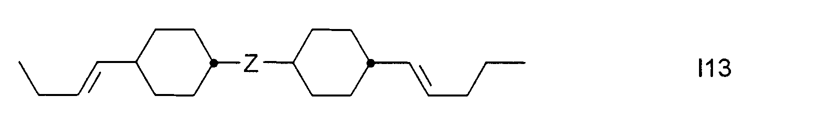

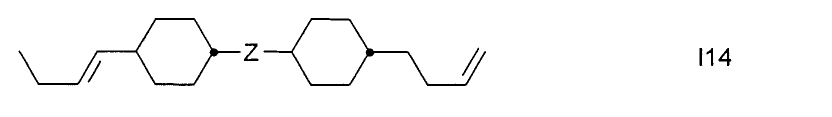

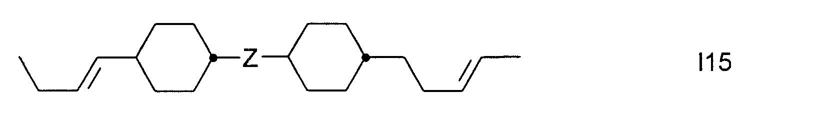

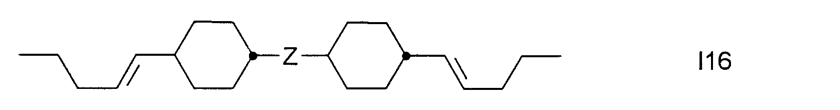

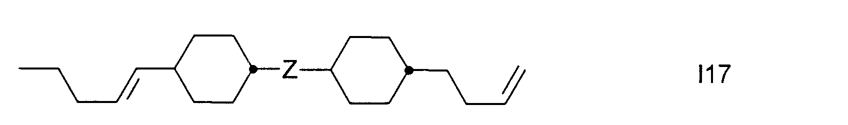

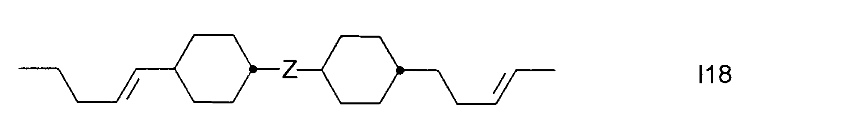

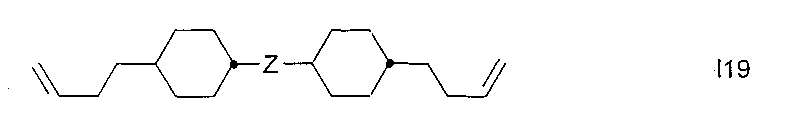

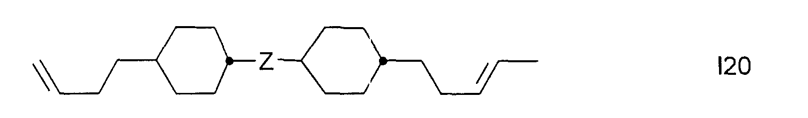

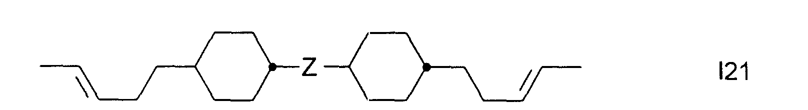

R¹ und R² bedeuten vorzugsweise 1E-Alkenyl oder 3E-Alkenyl mit 1-6 C-Atomen. Z ist vorzugsweise eine Einfachbindung oder eine -CH₂CH₂-Brücke.

Verbindungen der Formel I, die über für Polykondensationen geeignete Flügelgruppen R¹ und R² verfügen, eignen sich zur Darstellung flüssigkristalliner Polykondensate.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Cyclohexanringe trans-1,4-disubstituiert sind.

Unter Verbindungen der Formel I werden ebenfalls alle Verbindungen verstanden, worin C, H und O durch die entsprechenden Isotope ¹³C, ¹⁴C, D, T, ¹⁷O oder ¹⁸O ersetzt sind.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind.

Dabei kann man auch von an sich bekannten, hier nicht näher erwähnten Varianten Gebrauch machen.

Die erfindungsgemäßen Verbindungen können z.B. wie folgt hergestellt werden: 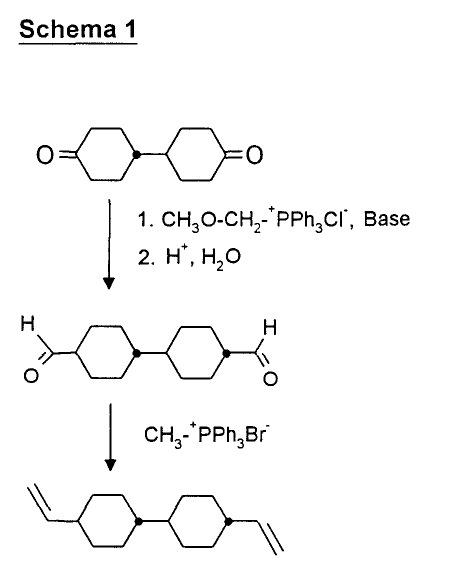 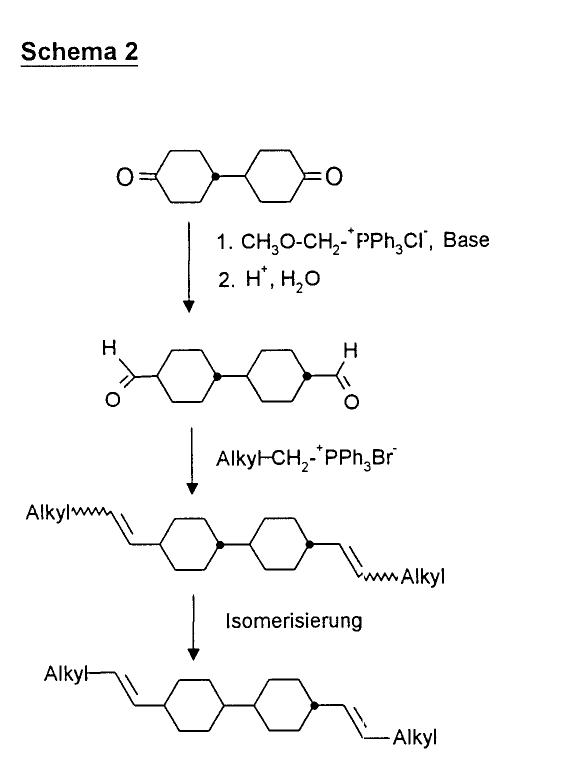 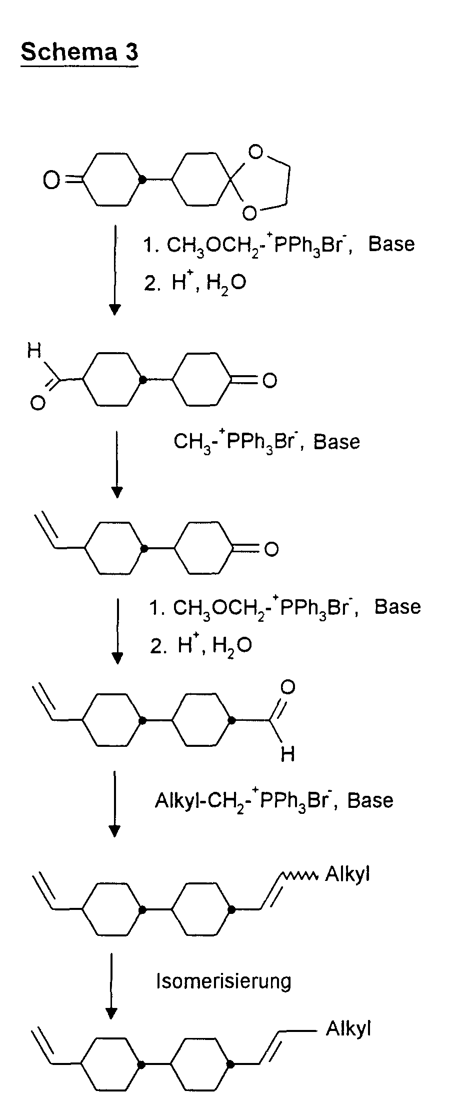 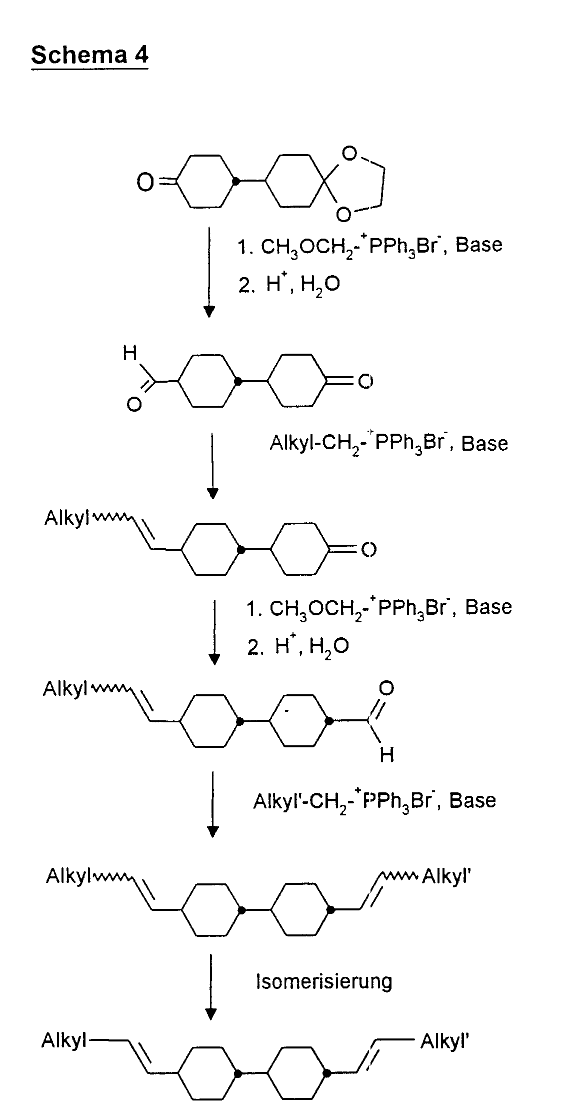 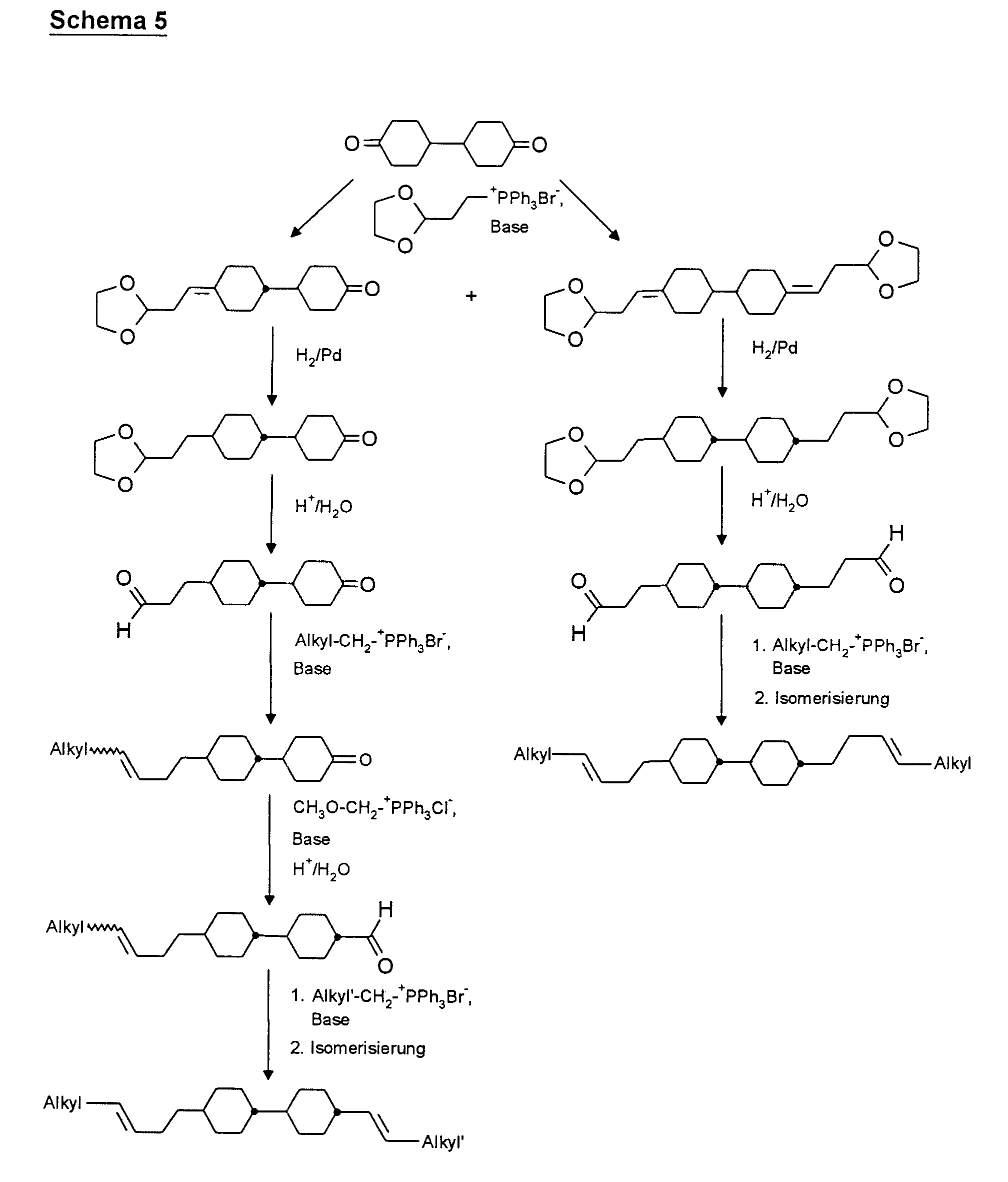 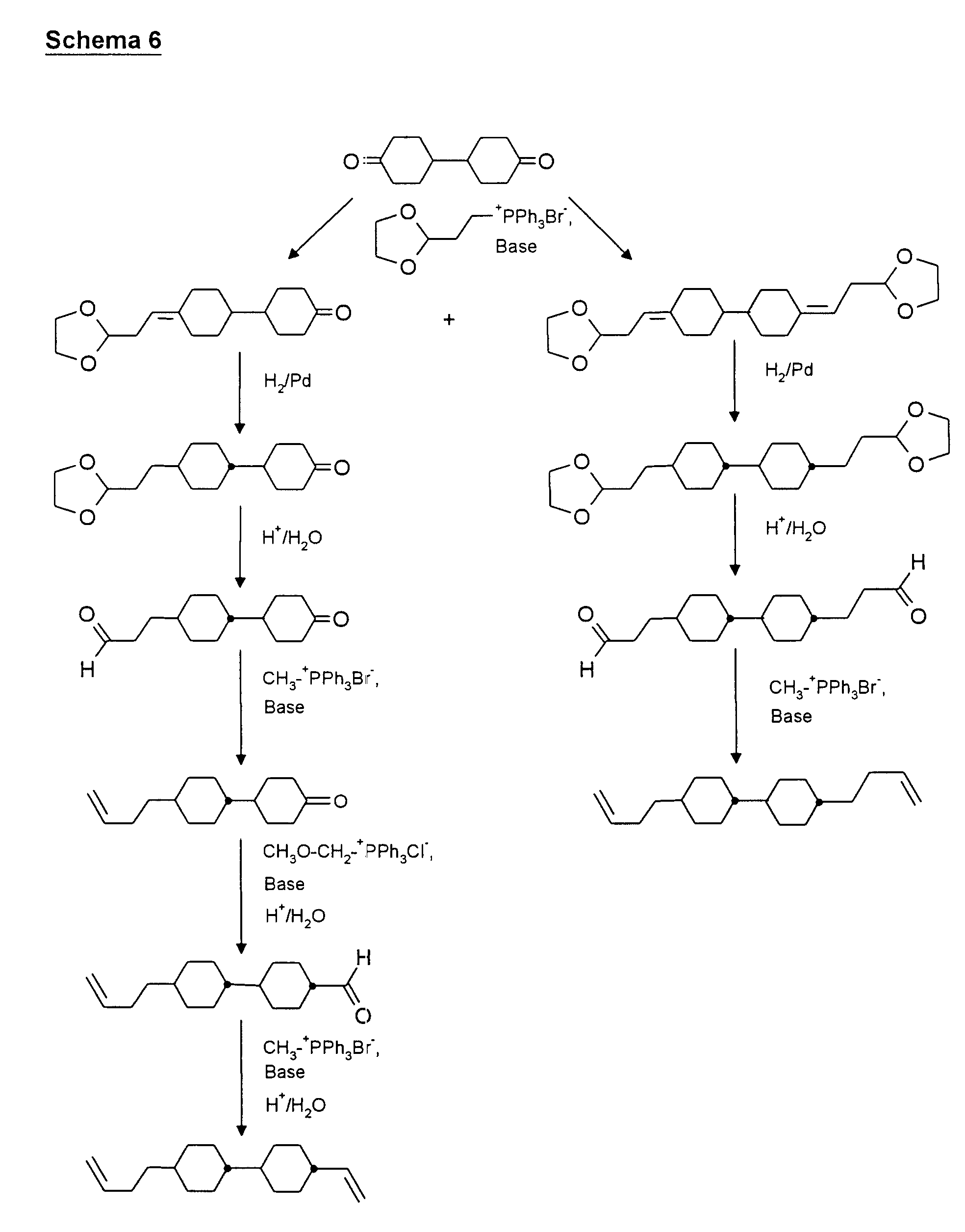 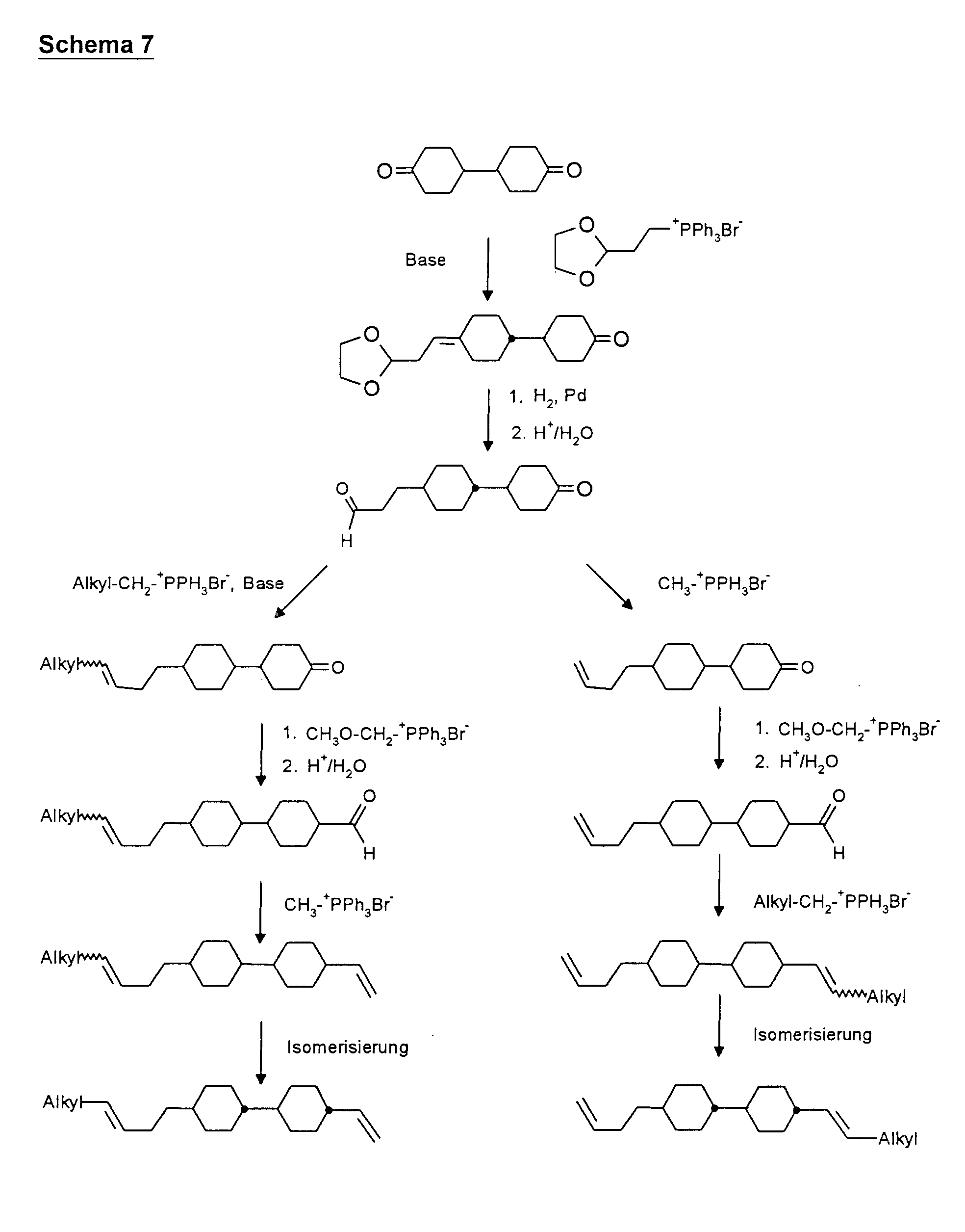 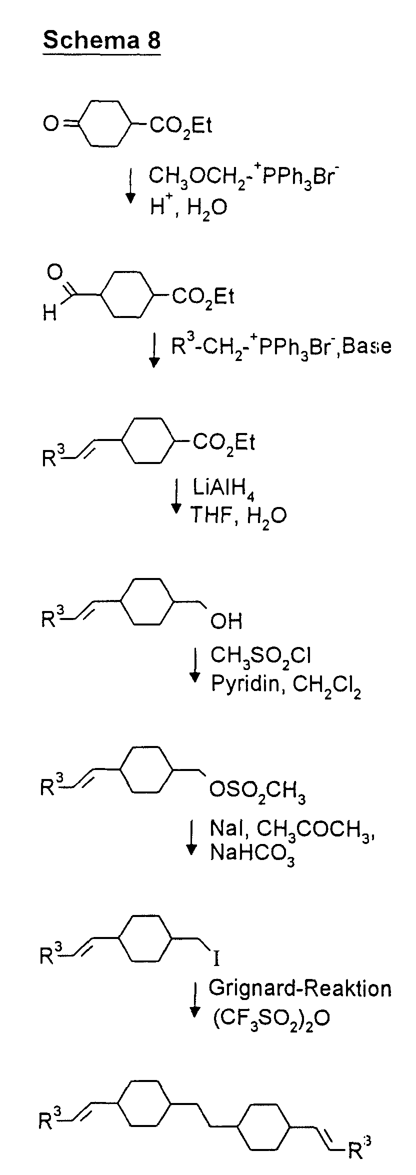 (R³ = H, CH₃, C₂H₅ oder n-C₃H₇) 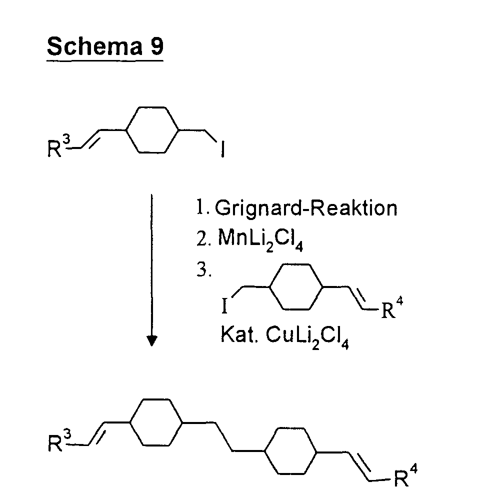 (R³, R⁴= H, CH₃, C₂H₅ oder n-C₃H₇)

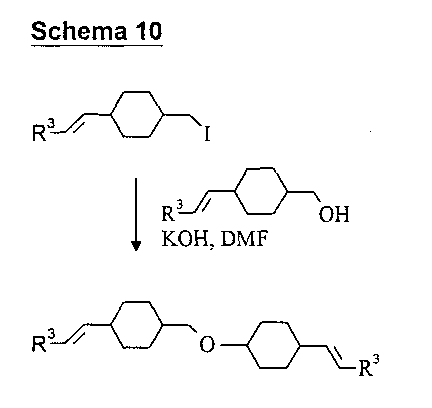

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexyl-ester, Phenyl- oder Cyclohexylester der Cyclohexylbenzoesäure, F'henyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Biscyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1 -Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R" 4

R'-L-C≡C-E-R'' 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢ CH₃₋₍ₖ₊ₗ₎ FₖClₗ, wobei i 0 oder 1 und k+l 1, 2 oder 3 sind; die Verbindungen, in denen R" diese Bedeutung hat, werden mit den Teilformeln 1b, 2b, 3b, 4b und 5b bezeichnet. Besonders bevorzugt sind solche Verbindungen der Teilformeln 1b, 2b, 3b, 4b und 5b, in denen R" die Bedeutung -F, -Cl, -NCS, -CF₃, -OCHF₂ oder -OCF₃ hat.

In den Verbindungen der Teilformeln 1b, 2b, 3b, 4b und 5b hat R' die bei den Verbindungen der Teilformeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN; diese Untergruppe wird im folgenden als Gruppe C bezeichnet und die Verbindungen dieser Untergruppe werden entsprechend mit Teilformeln 1c, 2c, 3c, 4c und 5 c beschrieben. In den Verbindungen der Teilformeln 1c, 2c, 3c, 4c und 5c hat R' die bei den Verbindungen der Teilformeln 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. Alle diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise

| | |
|---|---|
| Gruppe A | 0 bis 90 %, vorzugsweise 20 bis 90 %, insbesondere 30 bis 90 % |
| Gruppe B | 0 bis 80 %, vorzugsweise 10 bis 80 %, insbesondere 10 bis 65 % |
| Gruppe C | 0 bis 80 %, vorzugsweise 5 bis 80 %, insbesondere 5 bis 50 % |

wobei die Summe der Massenanteile der in den jeweiligen erfindungsgemäßen Medien enthaltenen Verbindungen aus den Gruppen A und/oder B und/oder C vorzugsweise 5 bis 90 % und insbesondere 10 bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/ R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen (n, m: 1-15). Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹ und L²:

| Code für R¹, R², L¹ und L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nF.F.F | CₙH₂ₙ₊₁ | F | F | F |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁-O-CₛH₂ₛ- | CN | H | H |
| nAm | CₙH₂ₙ₊₁ | COOCₘH₂ₘ₊₁ | H | H |
| nOCCF₂.F.F | CₙH₂ₙ₊₁ | OCH₂CF₂H | F | F |

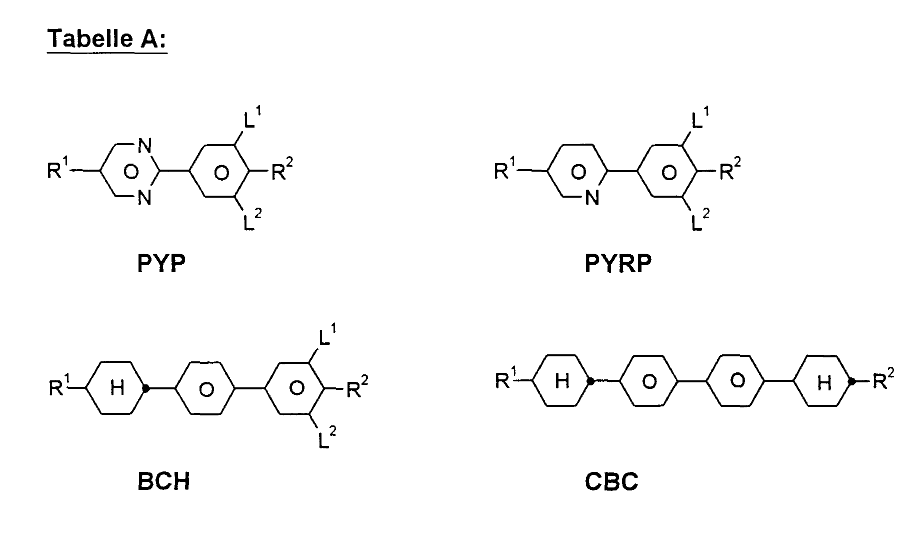

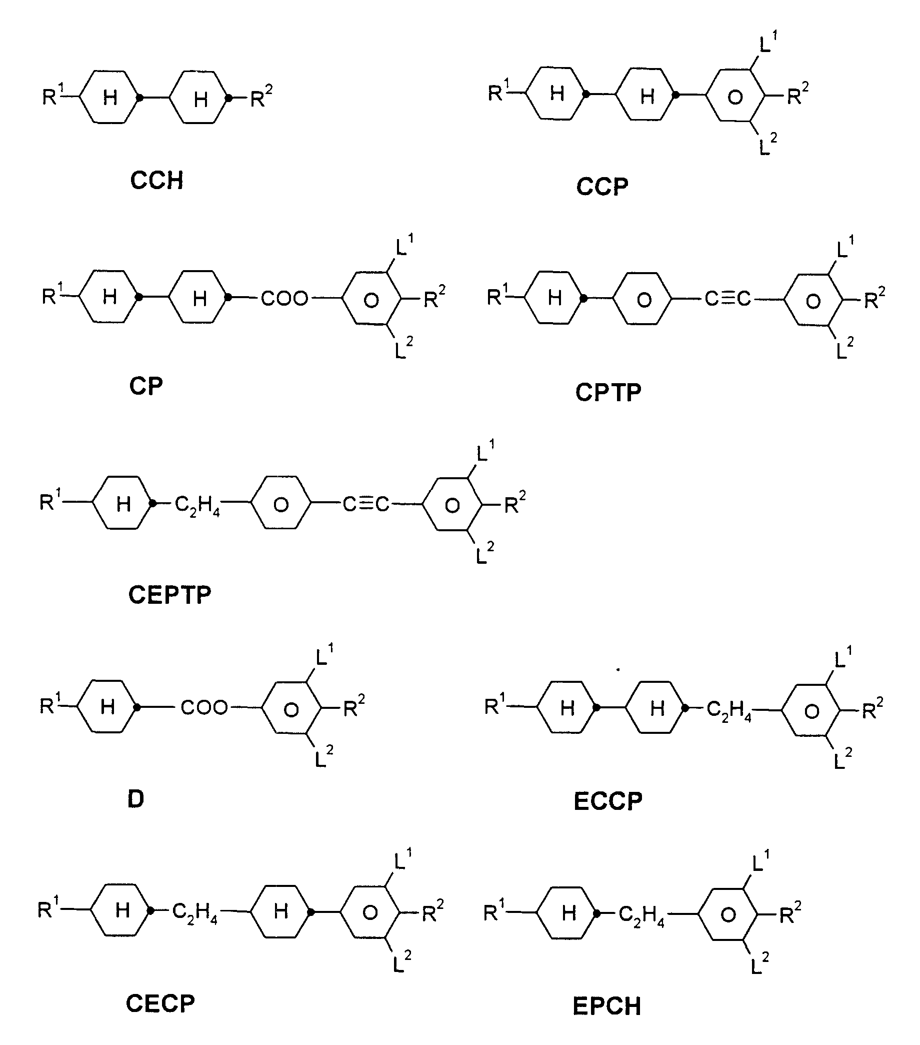

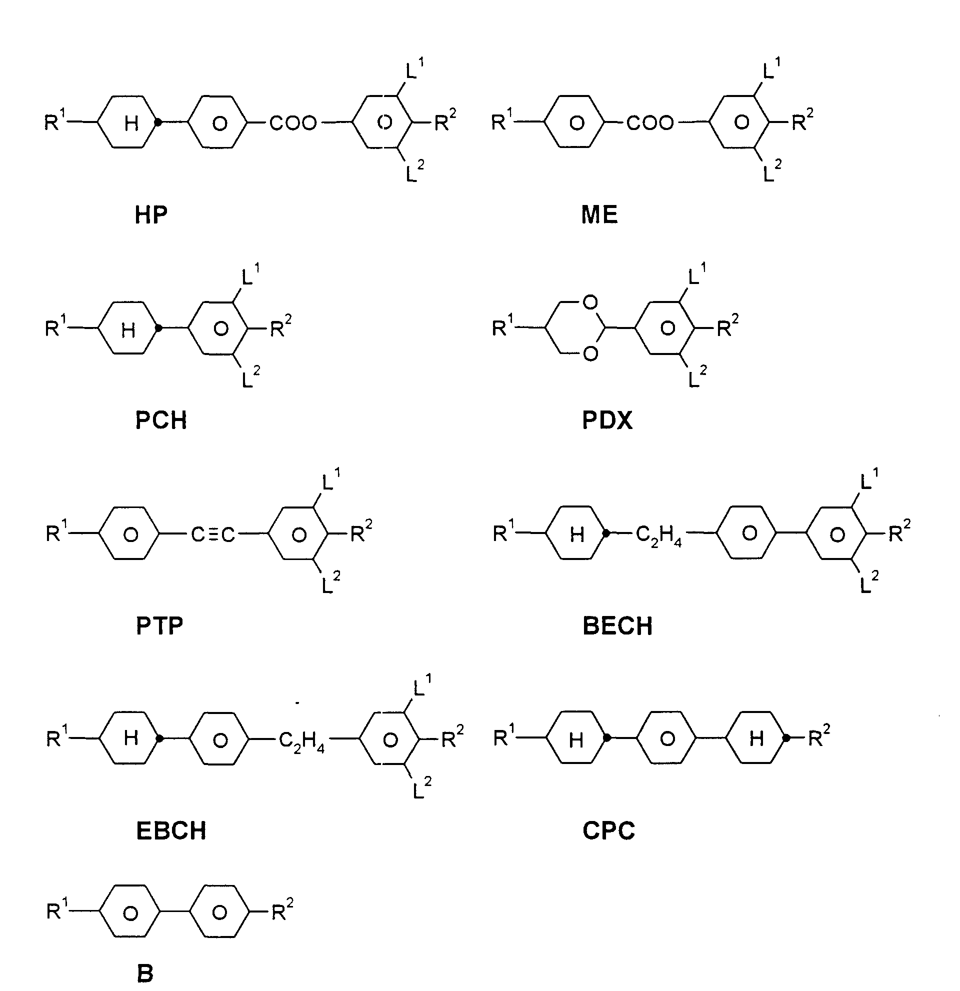

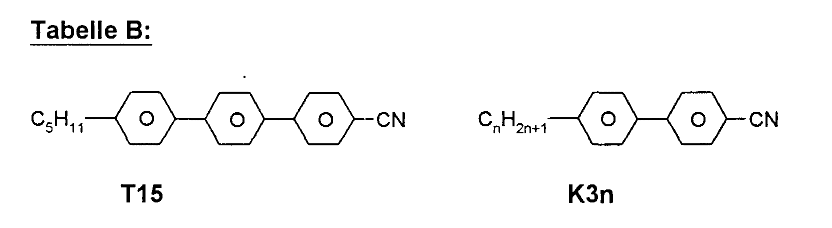

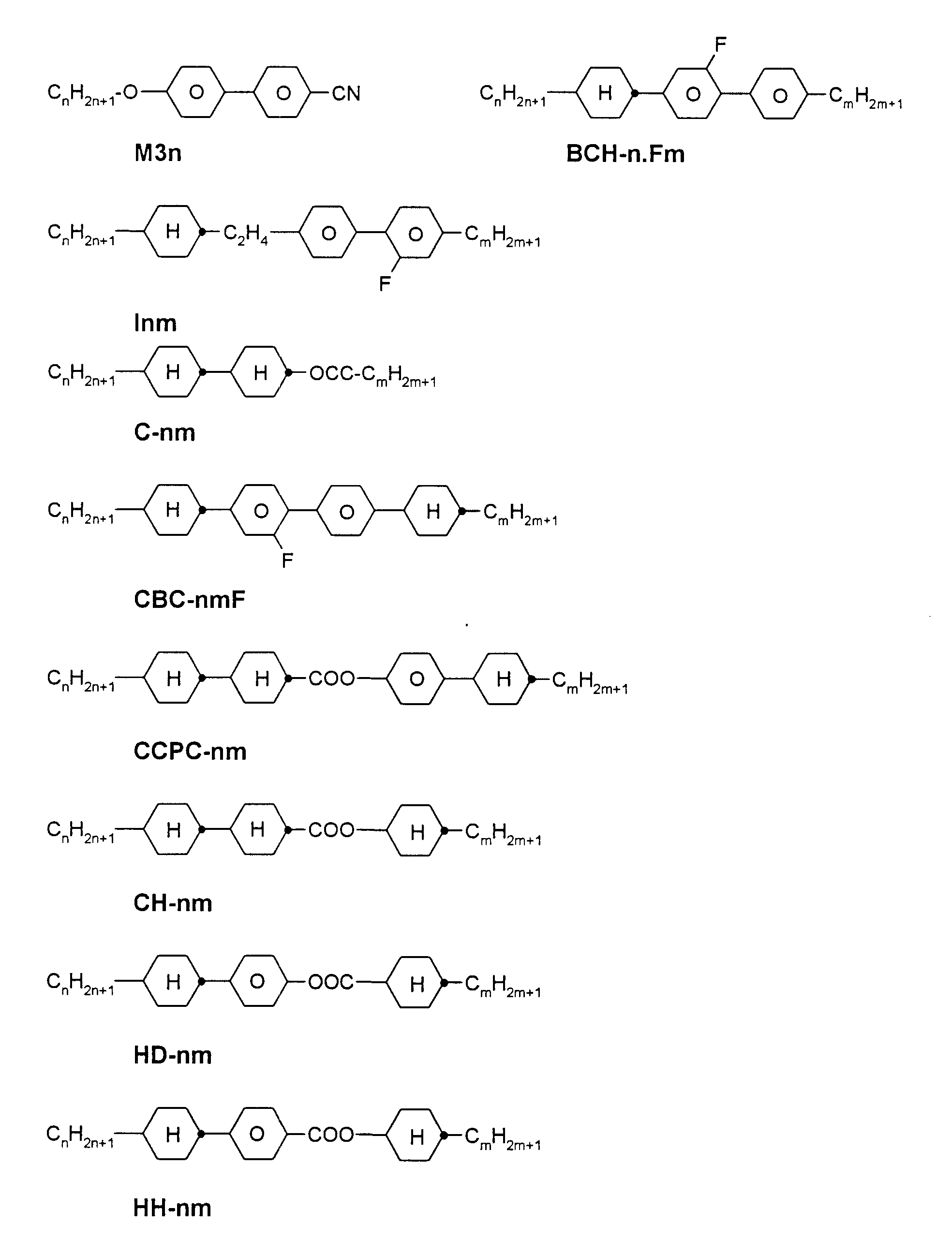

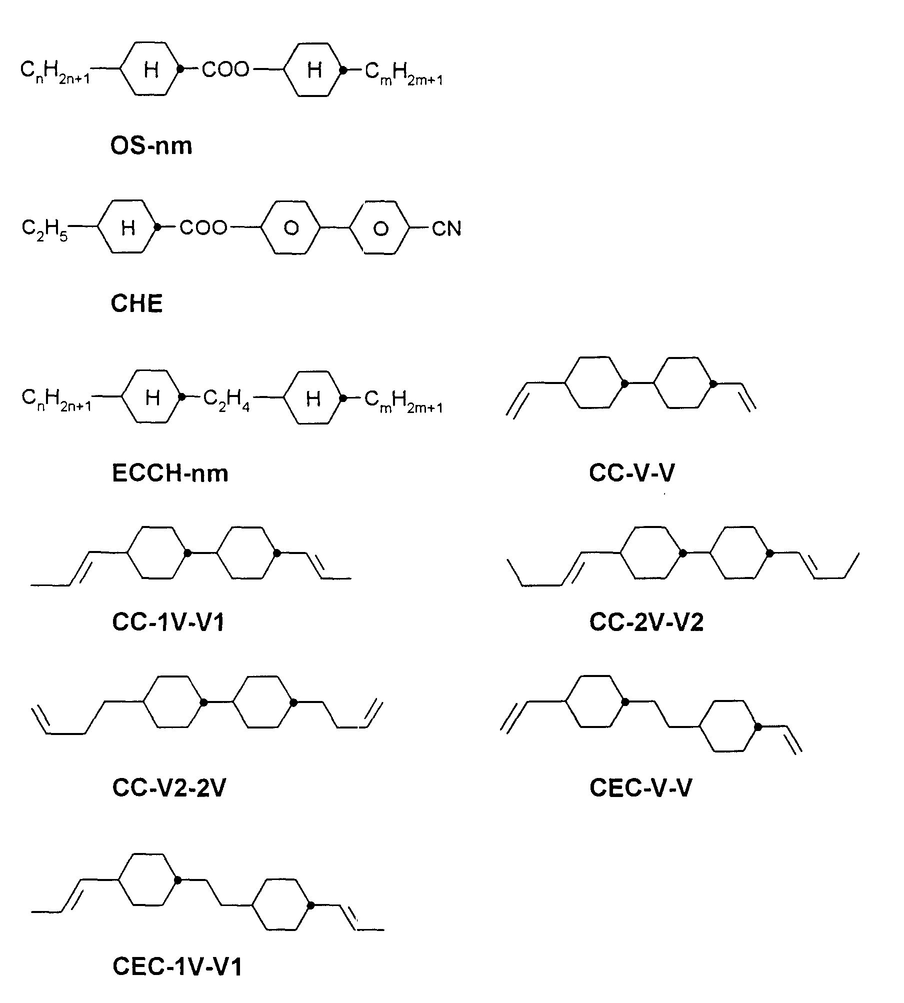

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I isotrope Phase. Die Angaben zwischen diesen Symbolen stellen die Übergangstemperaturen dar. Δn bedeutet optische Anisotropie (589 nm, 20 °C) und die Viskosität (mm²/sec) wurde bei 20 °C bestimmt.

"Übliche Aufarbeitung" bedeutet: man gibt gegebenenfalls Wasser hinzu, extrahiert mit Dichlormethan, Diethylether, Methyl-tert.Butylether oder Toluol, trennt ab, trocknet die organische Phase, dampft ein und reinigt das Produkt durch Destillation unter reduziertem Druck oder Kristallisation und/oder Chromatographie.

Folgende Abkürzungen werden verwendet:
- DMF: N,N-Dimethylformamid
- DMEU: 1,3-Dimethyl-2-imidazolidinon
- KOT: Kalium-tertiär-butanolat
- THF: Tetrahydrofuran
- pTsOH: p-Toluolsulfonsäure

### Beispiel 1

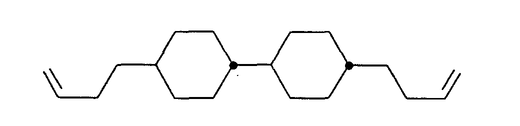

### Schritt 1.1

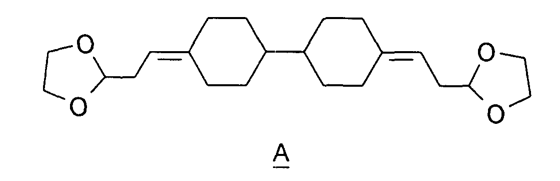

1,55 mol 2-(1,3-Dioxolan-2-yl-ethyl)-triphenylphosphoniumbromid werden in 800 ml abs. THF bei 0-5 °C suspendiert und unter N₂ mit 1,55 mol Kalium-tert.butylat gelöst in 500 ml abs. THF versetzt. Anschließend wird die Suspension mit 150 g Bicyclohexan-1,4-dion gelöst in 200 ml abs. THF bei 0-5 °C versetzt. Man läßt das Gemisch auf Raumtemperatur erwärmen, rührt über Nacht und hydrolysiert. Zuletzt wird wie üblich aufgearbeitet.

### Schritt 1.2

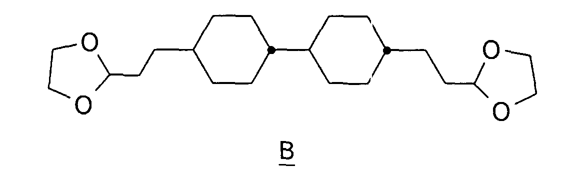

0,3 mol A werden in 1000 ml abs. THF in Gegenwart von Pd/C (5 %) hydriert. Nach beendeter Hydrierung wird abfiltriert, das Filtrat eingeengt und der Rückstand in Ethanol umkristallisiert.

### Schritt 1.3

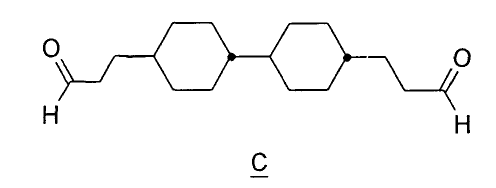

0,085 mol B werden in 600 ml Toluol gelöst und unter Rühren bei Raumtemperatur mit 250 ml Ameisensäure versetzt. Das Zweiphasengemisch läßt man über Nacht in einer Inertgasatmosphäre rühren. Nach der Zugabe von Wasser und Toluol wird wie üblich aufgearbeitet.

### Schritt 1.4

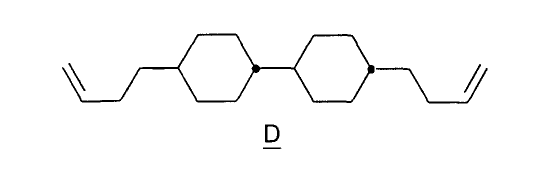

0,025 mol C und 0,052 mol Methyltriphenylphosphoniumbromid werden in 70 ml abs. THF gelöst und bei 0 °C in einer Argonatmosphäre vorgelegt und portionsweise mit 0,052 mol Kalium-tert.butylat gelöst in 80 ml THF bei -70 °C versetzt. Das Gemisch wird über Nacht bei Raumtemperatur gerührt. Man versetzt mit Wasser und 2N Salzsäure und arbeitet wie üblich auf. Das Produkt wird in 2-Propanol umkristallisiert. K 2 S_{B} 58 N 77,6 I; Δn = +0,052; Δε = -0,87; ν₂₀ = 6

Analog werden die folgenden Verbindungen der Formel 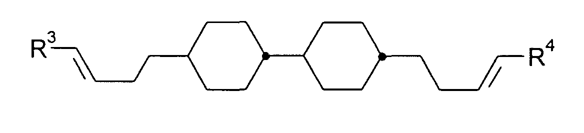 hergestellt:

| R³ | R⁴ |
|---|---|
| CH₃ | CH₃ |
| C₂H₅ | C₂H₅ |
| n-C₃H₇ | n-C₃H₇ |

### Beispiel 2

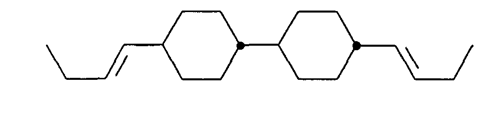

### Schritt 2.1

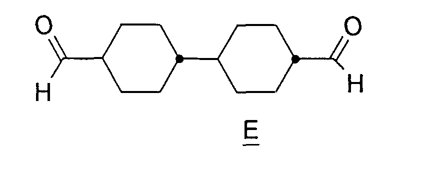

0,30 ml Bicyclohexan-1,4-dion und 0,7 mol Methoxymethyltriphenylphosphoniumchlorid werden in 1000 ml tert.Butylmethylether suspendiert. Das Gemisch wird auf 0-5 °C abgekühlt und mit 0,7 mol Kalium-tert.butylat gelöst in 500 ml abs. THF portionsweise versetzt. Die Reaktionslösung wird 5 Tage bei Raumtemperatur gerührt. Nach Zugabe von 10%iger HCI wird 3 h bei 50 °C nachgerührt. Das Gemisch wird mit Wasser und tert.Butylmethylether versetzt und zuletzt wie üblich aufgearbeitet.

### Schritt 2.2

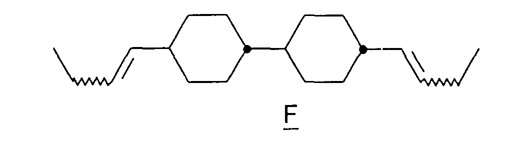

0,41 mol E und 0,82 mol Propyltriphenylphosphoniumbromid in 1000 ml abs. THF werden portionsweise mit Kalium-tert.butylat versetzt. Das Reaktionsgemisch wird über Nacht bei Raumtemperatur gerührt. Nach Zugabe von Wasser, 2N HCI und tert. Butylmethylether wird wie üblich aufgearbeitet.

### Schritt 2.3

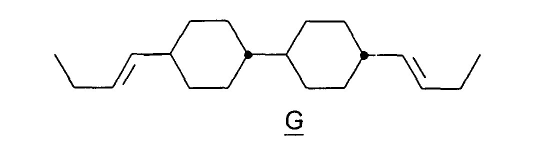

0,049 mol F, 80 ml Toluol, 2 g Benzolsulfonsäurenatriumsalz und 16 ml 1N HCI werden 2 Tage unter Rückfluß gekocht. Nach Zugabe von Wasser wird wie üblich aufgearbeitet. Das Produkt wird in 2-Propanol umkristallisiert. K 25 S_{B} 86 N 87 I; Δn = +0,063; Δε = -0,62

Analog werden die folgenden Verbindungen der Formel 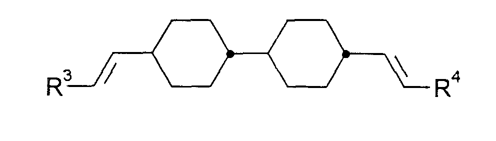 hergestellt:

| R³ | R⁴ | |
|---|---|---|
| H | H | K -9 N 20,4 I; Δn = +0,035; Δε = -1,93 |
| CH₃ | CH₃ | K 33 S_{B} 70 N 109,9 I; Δn = +0,081; Δε = -0,02 |
| C₂H₅ | C₂H₅ | |
| n-C₃H₇ | n-C₃H₇ | |

### Beispiel 3

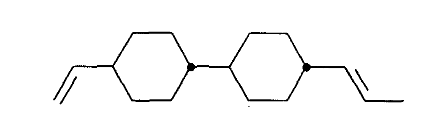

### Schritt 3.1

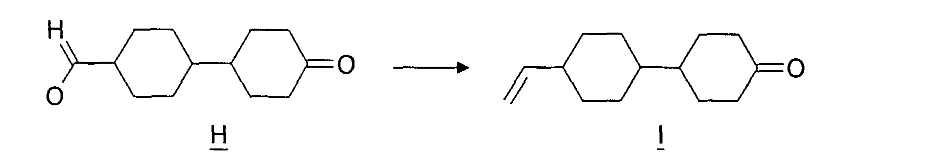

0,7 mol Methyltriphenylphosphoniumbromid in 1400 ml abs. THF werden bei Raumtemperatur tropfenweise mit 0,7 mol Kalium-tert.butylat gelöst in 500 ml abs. THF versetzt. Die Suspension wird 0,5 h gerührt und anschließend mit 0,68 mol H gelöst in 900 ml abs. THF versetzt. Man läßt 4 h rühren, versetzt mit Wasser, extrahiert mit Methyl-tert.butylether und arbeitet dann wie üblich auf.

### Schritt 3.2

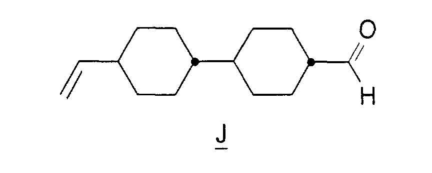

0,5 mol I und 0,6 mol Methoxytriphenylphosphoniumchlorid werden in 1000 ml abs. THF gelöst und bei ca. 10 °C mit einer Lösung bestehend aus 0,6 mol Kalium-tert.butylat in 200 ml abs. THF versetzt. Die Suspension wird über Nacht gerührt, mit Wasser und Methyl-tert.butylether versetzt und wie üblich aufgearbeitet.

### Schritt 3.3

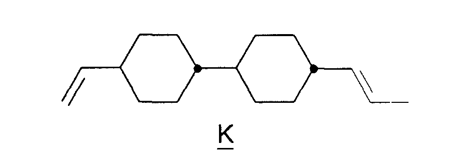

0,1 mol J und 0,1 mol Ethyltriphenylphosphoniumbromid werden in 200 ml abs. THF gelöst und portionsweise bei Raumtemperatur mit 0,1 mol Kalium-tert.butylat versetzt. Das Reaktionsgemisch wird über Nacht gerührt, mit Wasser versetzt, mit verd. HCI neutralisiert und wie üblich aufgearbeitet.

Für die cis/trans-Isomerisierung wird das Produkt (0,04 mol) in 80 ml Toluol gelöst, mit 3,7 g Benzolsulfinsäurenatriumsalz und 23 ml 1 N HCI versetzt und über Nacht am Rückfluß gekocht. Man läßt die Reaktionslösung auf Raumtemperatur abkühlen, versetzt mit Bicarbonat-Lösung und arbeitet zuletzt wie üblich auf. K-16 N 54,3 I; Δε = - 0,92; Δn = +0,060.

Analog werden die folgenden Verbindungen der Formel 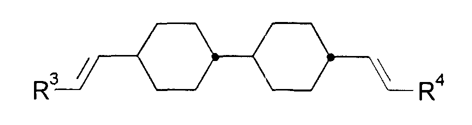 hergestellt:

| R³ | R⁴ | |
|---|---|---|
| H | CH₃ | |
| H | C₂H₅ | K -28 S_{B} 30 N 39,1 I; Δn = +0,049; Δε = -1,12 |
| H | n-C₃H₇ | K -32 S_{B} 49 N 54,8 I; Δn = +0,056; Δε = -0,99 |
| CH₃ | C₂H₅ | K 13 S_{B} 29 N 78,9 I; Δn = +0,065; Δε = -0,6 |
| CH₃ | n-C₃H₇ | K 7 S_{B} 62 N 100,8 I; Δn = +0,074; Δε = -0,2 |
| C₂H₅ | n-C₃H₇ | |

### Beispiel 4

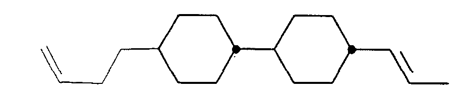

### Schritt 4.1

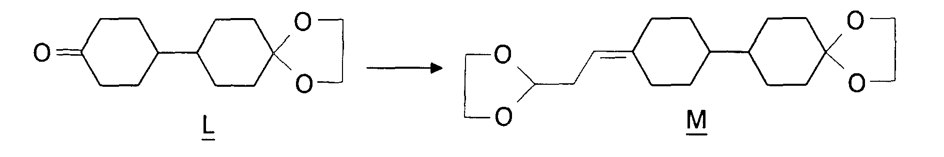

1,15 mol Dioxolan-ethyltriphenylphosphoniumbromid in 1,8 I abs. THF werden tropfenweise mit 1,15 mol Kalium-tert.butylat gelöst in 400 ml abs. THF bei ca. 10 °C versetzt. Die Suspension wird 15 Min. gerührt und bei 10 °C mit 1,05 mol L gelöst in 700 ml abs. THF versetzt. Das Reaktionsgemisch wird über Nacht gerührt, mit Wasser versetzt und verd. HCI neutralisiert. Nach der Extraktion mit Methyl-tert.butylether wird wie üblich aufgearbeitet.

### Schritt 4.2

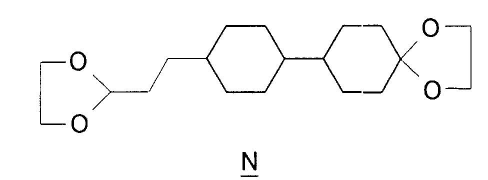

0,65 mol M gelöst in 2,5 l THF werden in Gegenwart von 30 g Pd/C hydriert. Nach beendeter Reaktion wird der Katalysator abfiltriert und das Filtrat am Rotavapor eingeengt.

### Schritt 4.3

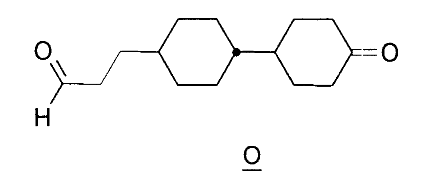

0,7 mol N, 1000 ml Ameisensäure und 3000 ml Toluol werden bei Raumtemperatur über Nacht gerührt. Die Reaktionslösung wird mit Wasser versetzt und wie üblich aufgearbeitet.

### Schritt 4.4

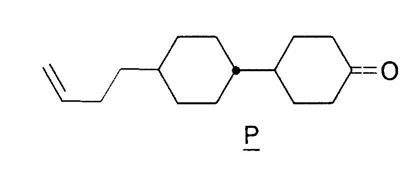

0,5 mol Methyltriphenylphosphoniumbromid in 1200 ml abs. THF werden bei Raumtemperatur tropfenweise mit einer Lösung bestehend aus 0,5 mol Kalium-tert.butylat in 300 ml abs. THF versetzt. Das Reaktionsgemisch wird 0,5 h gerührt und anschließend mit 0,5 mol O gelöst in 500 ml abs. THF versetzt. Nach 4 h Rühren bei Raumtemperatur wird das Reaktionsgemisch mit Wasser verdünnt, mit Methyl-tert.butylether extrahiert und wie üblich aufgearbeitet.

### Schritt 4.5

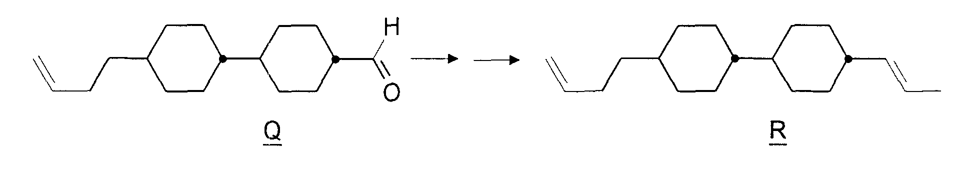

Analog Schritt 3.2 und Schritt 3.3 werden 0,09 mol P zunächst in den Aldehyd Q und anschließend in das Bisalkenyl R überführt. Zuletzt erfolgt die cis/trans-Isomerisierung.
K 4 S_{B} 17 N 84 l; Δn = +0,066; Δε = -0,38

Analog werden die folgenden Verbindungen der Formel 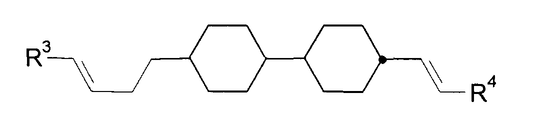 hergestellt:

| R ³ | R ⁴ | |
|---|---|---|
| H | H | K -48 S _{B} 1 N 49 I; Δn = +0,045; Δε = -1,14; ν ₂₀ = 4 |
| H | C₂H₄ | K -4 S _{B} 77 N 78,8 I; Δn = +0,061; Δε = -0,66 |
| H | n-C₃H₇ | |
| C₂H₅ | H | |
| C₂H₅ | CH₃ | |
| C₂H₅ | C₂H₅ | |
| C₂H₅ | n-C₃H₇ | |
| n-C₃H₇ | H | |
| n-C₃H₇ | CH₃ | |
| n-C₃H₇ | C₂H₅ | |
| n-C₃H₇ | n-C₃H₇ | |

### Beispiel 5

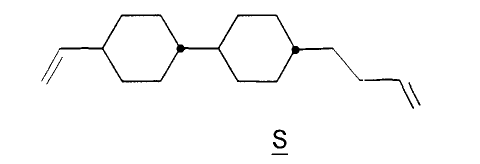

Analog der Schritte 4.1 bis 4.5 wird das Zwischenprodukt P mit 2-(1,3-Dioxolan-2-yl-methyl)-triphenylphosphoniumbromid umgesetzt, anschließend hydriert, in den Aldehyd und zuletzt in die Bisalkenylverbindung S überführt. Zuletzt erfolgt die cis/trans-Isomerisierung K 29 S_{B} 57 I; Δn = + 0,027; Δε= - 1,8

### Beispiel 6

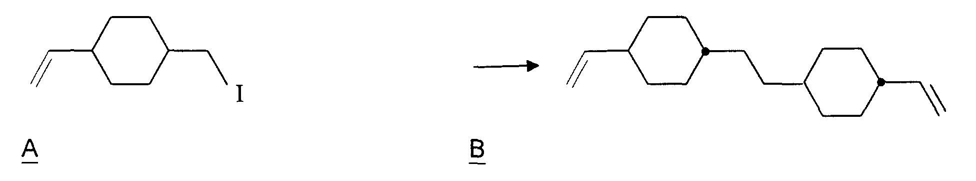

37 mmol A (hergestellt gemäß Schema 8) in 100 ml Diethylether werden mit 38,5 mmol Magnesium vorgelegt und mit einem Tropfen Methyliodid versetzt. Nach beendeter Grignard-Reaktion läßt man das Reaktionsgemisch auf Raumtemperatur abkühlen und versetzt portionsweise mit 7 mmol Trifluormethansulfonsäureanhydrid gelöst in 20 ml Diethylether.

Das Reaktionsgemisch wird 0,5 h in der Siedehitze gerührt. Nach der Hydrolyse wird wie üblich aufgearbeitet. Das Rohprodukt wird mit n-Hexan über Kieselgel eluiert und zuletzt aus Hexan/Ethanol (1:5) umkristallisiert. K 25 N 25,7 I; Δn = +0,031; Δε = -2,12; ν₂₀= 5.

Analog werden die folgenden Verbindungen der Formel 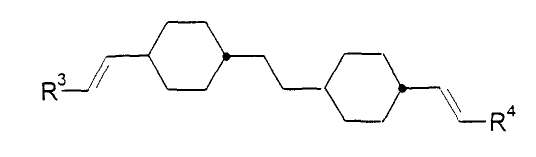 hergestellt:

| R³ | R⁴ | |
|---|---|---|
| CH₃ | CH₃ | K-1 S 88 N 88, 9 I;Δn = 0,071; Δε = 0,16 |
| C₂H₅ | C₂H₅ | |
| n-C₃H₇ | n-C₃H₇ | |

### Beispiel 7

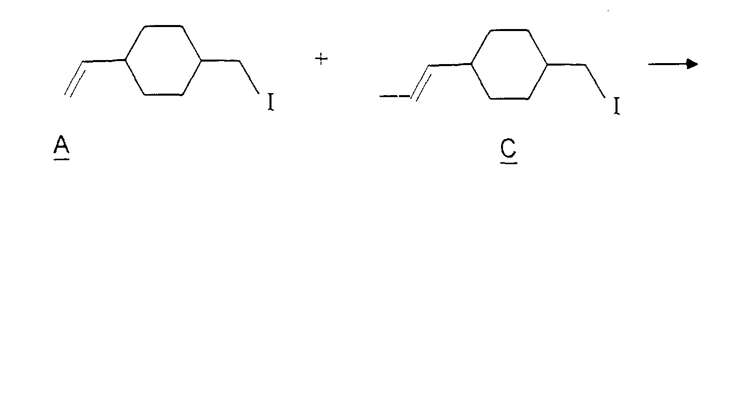

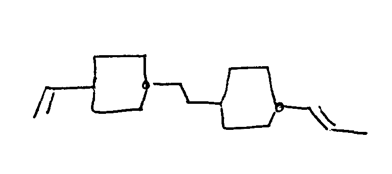

0,1 mol A werden analog Beispiel 6 in die Grignardverbindung überführt und diese Lösung wird bei -10 °C zu einer Lösung von 0,1 mol MnLi₂Cl₄ in THF (hergestellt aus MnCl₂ und LiCI) getropft. Anschließend setzt man 0,1 mol C und 0,002 mol CuLi₂Cl₄ (gelöst in THF) zu und rührt 3 h bei Raumtemperatur. Zuletzt wird wie üblich aufgearbeitet.

Analog werden die folgenden Verbindungen der Formel 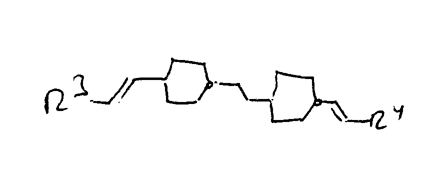 hergestellt:

| R³ | R⁴ |
|---|---|
| H | H |
| H | C₂H₅ |
| H | n-C₃H₇ |
| CH₃ | C₂H₅ |
| CH₃ | n-C₃H₇ |
| C₂H₅ | C₂H₅ |
| C₂H₅ | n-C₃H₇ |
| n-C₃H₇ | n-C₃H₇ |

### Vergleichsbeispiel 1

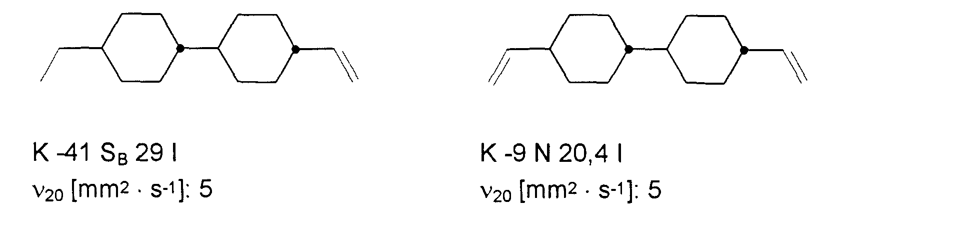

### Vergleichsbeispiel 2

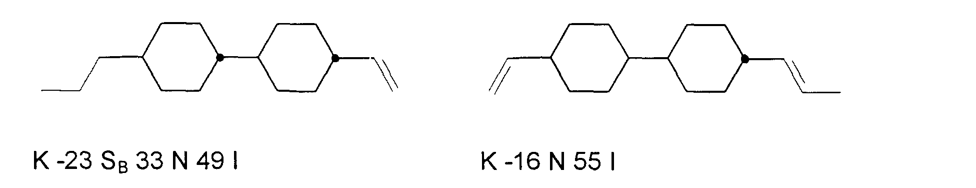

### Vergleichsbeispiel 3

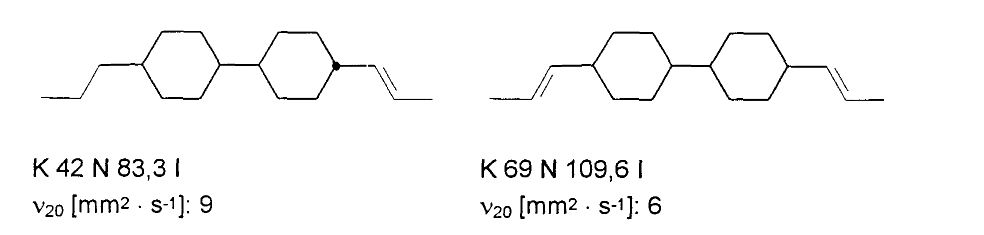

### Vergleichsbeispiel 4

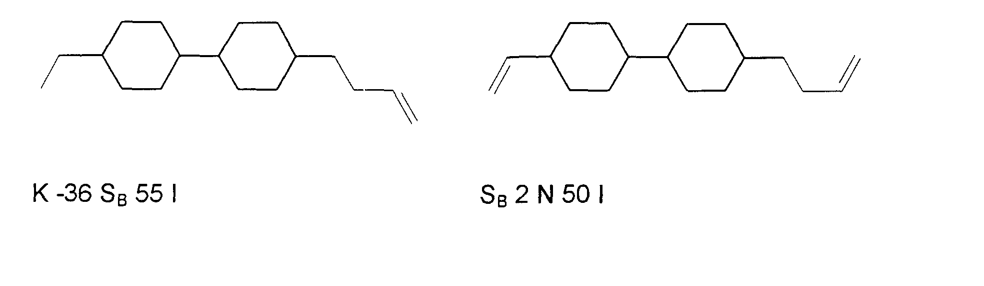

### Vergleichsbeispiel 5

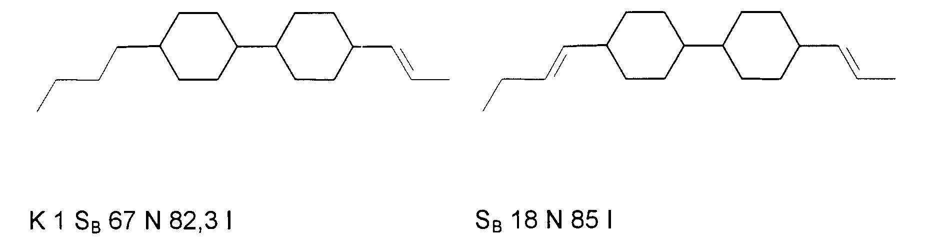

Die Vergleichsbeispiele 1-5 zeigen, daß die erfindungsgemäßen Dialkenylverbindungen eine geringere Tendenz zu smektischen Phasen, einen höheren Klärpunkt und eine niedrigere Viskosität aufweisen als die entsprechenden Monoalkenylverbindungen.

## Patentansprüche

1. Bisalkenylbicyclohexane der Formel I, 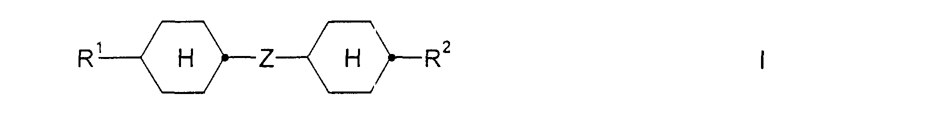 worin
R¹ und R² jeweils einen Alkenylrest mit 2 bis 7 C-Atomen, und
Z -CH₂CH₂-, -OCH₂-, -CH₂O- oder eine Einfachbindung
bedeuten.

2. Bisalkenylbicyclohexane nach Anspruch 1, **dadurch gekennzeichnet**, daß R¹ und R² jeweils unabhängig voneinander 1-E-Alkenyl oder 3-E-Alkenyl bedeuten.

3. Verbindungen nach Anspruch 1, **dadurch gekennzeichnet**, daß Z -CH₂CH₂- oder eine Einfachbindung bedeutet.

4. Verbindungen der Formel IA 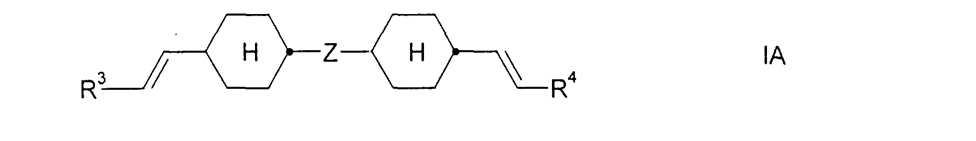 worin
R³ und R⁴ jeweils unabhängig voneinander H oder einen Alkylrest mit 1 bis 5 C-Atomen, und
Z -CH₂CH₂-, -OCH₂-, -CH₂O- oder eine Einfachbindung
bedeuten.

5. Verbindungen der Formel IB 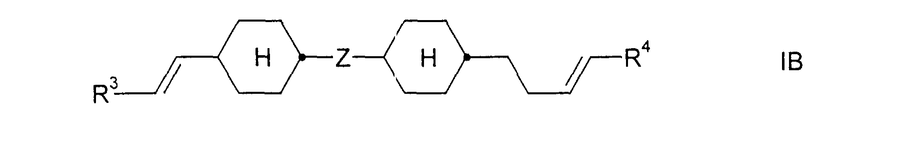 worin
R³ und R⁴ jeweils unabhängig voneinander H oder einen Alkylrest mit 1 bis 5 C-Atomen, und
Z -CH₂CH₂-, -OCH₂-, -CH₂O- oder eine Einfachbindung
bedeuten.

6. Verbindungen der Formel IC 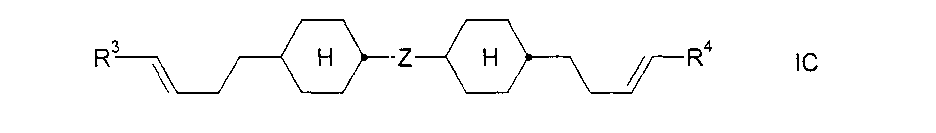 worin
R³ und R⁴ jeweils unabhängig voneinander H oder einen Alkylrest mit 1 bis 5 C-Atomen, und
Z -CH₂CH₂-, -OCH₂-, -CH₂O- oder eine Einfachbindung
bedeuten.

7. Verbindungen nach Anspruch 4, worin Z eine -CH₂CH₂-Brücke bedeutet.

8. Verbindung der Formel

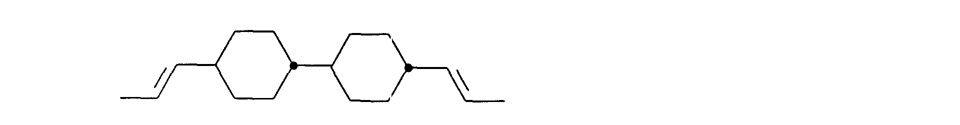

9. Verwendung von Verbindungen der Formel I als Komponenten flüssigkristalliner Medien.

10. Flüssigkristallines Medium mit mindestens zwei flüssigkristallinen Komponenten, **dadurch gekennzeichnet**, daß es mindestens eine Verbindung der Formel I enthält.

11. Flüssigkristall-Anzeigeelement, **dadurch gekennzeichnet**, daß es ein flüssigkristallines Medium nach Anspruch 10 enthält.

12. Elektrooptisches Anzeigeelement, **dadurch gekennzeichnet**, daß es als Dielektrikum ein flüssigkristallines Medium nach Anspruch 10 enthält.

## Claims

1. Bisalkenylbicyclohexanes of the formula I 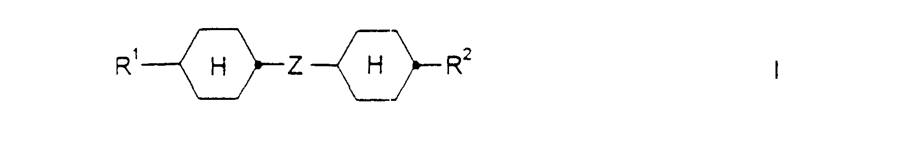 in which
R¹ and R² are each an alkenyl radical having 2 to 7 carbon atoms, and
Z is -CH₂CH₂-, -OCH₂-, -CH₂O- or a single bond.

2. Bisalkenyl bicyclohexanes according to Claim 1, **characterized in that** R¹ and R² are each, independently of one another, 1E-alkenyl or 3E-alkenyl.

3. Compounds according to Claim 1, **characterized in that** Z is -CH₂CH₂- or a single bond.

4. Compounds of the formula IA 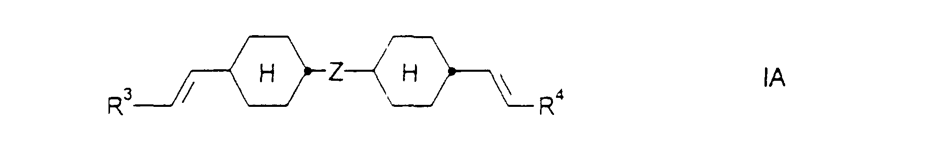 in which
R³ and R⁴ are each, independently of one another, H or an alkyl radical having 1 to 5 carbon atoms, and
Z is -CH₂CH₂-, -OCH₂-, -CH₂O- or a single bond.

5. Compounds of the formula IB 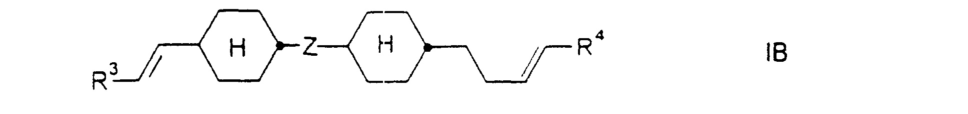 in which
R³ and R⁴ are each, independently of one another, H or an alkyl radical having 1 to 5 carbon atoms, and
Z is -CH₂CH₂-, -OCH₂-, -CH₂O- or a single bond.

6. Compounds of the formula IC 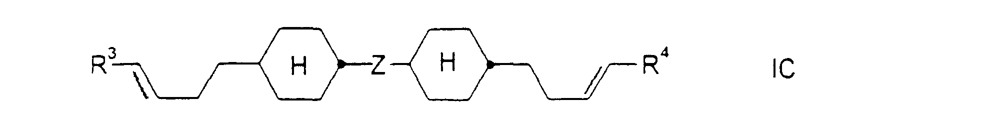 in which
R³ and R⁴ are each, independently of one another, H or an alkyl radical having 1 to 5 carbon atoms, and
Z is -CH₂CH₂-, -OCH₂-, -CH₂O- or a single bond.

7. Compounds according to Claim 4, in which Z is a -CH₂CH₂- bridge.

8. Compound of the formula

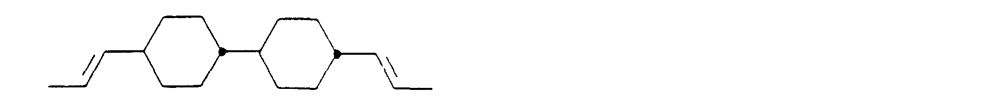

9. Use of compounds of the formula I as components of liquid-crystalline media.

10. Liquid-crystalline medium having at least two liquid-crystalline components, **characterized in that** it includes at least one compound of the formula I.

11. Liquid-crystal display element, **characterized in that** it contains a liquid-crystalline medium according to Claim 10.

12. Electro-optical display elements, **characterized in that** it contains, as dielectric, a liquid-crystalline medium according to Claim 10.

## Revendications

1. Bis-alcènyle-bicyclohexane de la formule I, 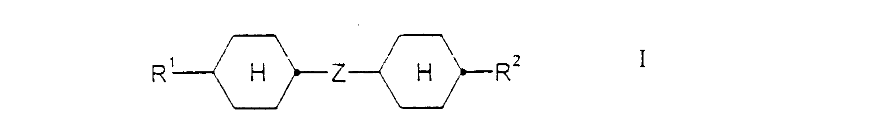 où
R¹ et R² représentent respectivement un radical alkyle doté de 2 à 7 atomes de C, et
Z représente -CH₂CH₂-, -OCH₂-, -CH₂O- ou une liaison simple.

2. Bis-alcènyle-bicyclohexane selon la revendication 1, **caractérisé en ce que** R¹ et R² représentent respectivement indépendamment l'un de l'autre 1-E-alcènyle ou 3-E-alcènyle.

3. Composés selon la revendication 1, **caractérisés en ce que** Z représente-CH₂CH₂- ou une liaison simple.

4. Composés de la formule IA 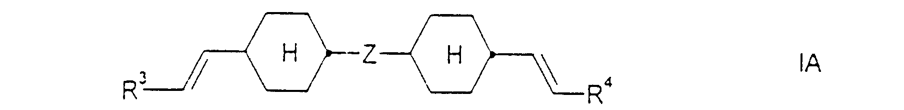 où
R³ et R⁴ représentent respectivement indépendamment l'un de l'autre H ou un radical alkyle doté de 1 à 5 atomes de C, et
Z représente -CH₂CH₂-, -OCH₂-, -CH₂O- ou une liaison simple.

5. Composés de la formule IB 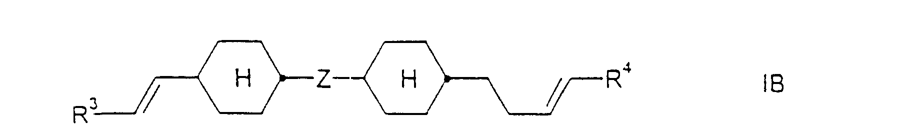 ou
R³ et R⁴ représentent respectivement indépendamment l'un de l'autre H ou un radical alkyle doté de 1 à 5 atomes de C, et
Z représente -CH₂CH₂-, -OCH₂-, -CH₂O- ou une liaison simple.

6. Composés de la formule IC 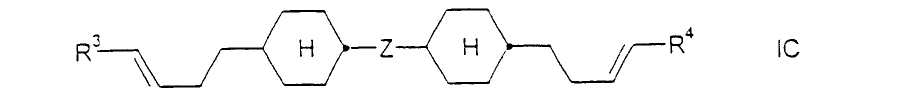 où
R³ et R⁴ représentent respectivement indépendamment l'un de l'autre H ou un radical alkyle doté de 1 à 5 atomes de C, et
Z représente -CH₂CH₂-, -OCH₂-, -CH₂O- ou une liaison simple.

7. Composés selon la revendication 4, où Z représente un pont -CH₂CH₂-.

8. Composé de la formule

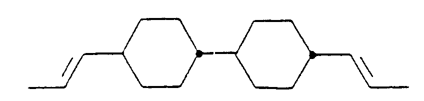

9. Utilisation des composés de la formule I comme composants de milieux cristaux liquides.

10. Milieu cristal liquide avec au moins deux composants cristaux liquides, **caractérisé en ce qu**'il contient au moins un composé de la formule I.

11. Elément d'affichage cristal liquide, **caractérisé en ce qu**'il contient un milieu cristal liquide selon la revendication 10.

12. Elément d'affichage électro-optique, **caractérisé en ce qu**'il contient à titre de diélectrique un milieu cristal liquide selon la revendication 10.
